# EUROPEAN PATENT SPECIFICATION

(11) **EP 2 857 427 B1**
(45) Date of publication and mention of the grant of the patent: **08.01.2020**
(21) Application number: 13797206.3
(22) Date of filing: 31.05.2013
(51) Int. Cl.: C08G 18/22, C07D 487/08, C08G 18/18, C07F 15/02

(54) **CATALYST COMPOSITION FOR POLYURETHANE RESIN PRODUCTION AND METHOD FOR PRODUCING POLYURETHANE RESIN USING SAME**
KATALYSATORZUSAMMENSETZUNG ZUR POLYURETHANHARZHERSTELLUNG UND VERFAHREN ZUR HERSTELLUNG VON POLYURETHANHARZ DAMIT
COMPOSITION DE CATALYSEUR POUR UNE FABRICATION DE RÉSINE DE POLYURÉTHANE ET PROCÉDÉ DE FABRICATION DE RÉSINE DE POLYURÉTHANE L'UTILISANT

(30) Priority: 31.05.2012 JP 2012124777
(43) Date of publication of application: 08.04.2015
(73) Proprietor: Tosoh Corporation, Yamaguchi 746-8501 (JP)
(72) Inventor: MAEHAMA, Seiji, Shunan-shi Yamaguchi 746-8501 (JP)
(74) Representative: Vigand, Philippe
(86) International application number: PCT/JP2013/065250
(87) International publication number: WO 2013/180290

(56) References cited:
- EP-A1- 1 460 094
- JP-A- 2006 206 781
- JP-A- 2007 045 980
- JP-A- 2010 106 192
- JP-A- 2011 111 537
- JP-A- 2011 219 605
- JP-A- 2012 144 697

## Description

### TECHNICAL FIELD

The present invention relates to a catalyst composition for polyurethane resin production and a method for producing a polyurethane resin using the same. The catalyst composition of the present invention is excellent in the reactivity and the storage stability, and it is thereby possible to produce a polyurethane resin with good productivity.

### BACKGROUND ART

A polyurethane resin is produced by reacting a polyol with an organic polyisocyanate in the presence of a catalyst and, as the case requires, additives such as a blowing agent, a surfactant, a cross-linking agent, etc., and it is widely utilized, for example, as a coating material, an adhesive, an elastomer, a sealant, a rigid or flexible polyurethane foam, etc.

As a catalyst for the production of a polyurethane resin, a tertiary amine compound or a metal compound is used. A tertiary amine compound has a function not only to accelerate a reaction (gelling reaction) to form an urethane bond from a polyol and an organic polyisocyanate, but also to accelerate a reaction of water with an organic polyisocyanate to form a carbon dioxide gas (blowing reaction), and therefore, it may suitably be used for the production of a water blown polyurethane foam.

On the other hand, a metal compound such as a tin or lead compound mainly accelerates the gelling reaction and therefore is used as a strong gelling catalyst for a urethane foam or non-foamed urethane for e.g. an elastomer or an adhesive.

In recent years, along with an increased concern about environment, use of a highly toxic tin or lead compound tends to be discouraged. In some applications, a tertiary amine compound is used as a substitute catalyst. However, in an application where a particularly high gelling activity or a high reactivity to an aliphatic isocyanate is required, substitution by a tertiary amine compound is difficult, and a demand for a low toxic metal catalyst still continues to exist.

Heretofore, iron, titanium, zirconium and bismuth compounds have been studied as metal catalysts which are lower in toxicity than a tin or lead compound. Among them, an iron compound, particularly an acetyl acetonate salt of iron, is known from long ago as a catalyst whereby the gelling reaction can be proceeded very efficiently (e.g. Patent Documents 1 to 3).

However, because of various problems, their practical use has been limited.

For example, with respect to a conventional low toxic metal catalyst containing an iron compound, it is known that the activity decreases as time passes in the presence of a small amount of water. Therefore, there has been a serious problem such that when it is stored as added to a polyol component, its reactivity decreases due to remaining water. Further, in a water blown foam using, as a blowing agent, carbon dioxide gas formed by a reaction of water and an isocyanate, there has been a problem such that the initial activity is low, since the reactivity decreases immediately after contact of the catalyst with water. Furthermore, it has been extremely difficult to stably store it in a polyol premix containing water.

Further, an acetylacetone salt, iron chloride or iron carboxylate commonly used as an iron compound, has had a problem such that it is poor in the operation efficiency, since it has a low solubility in a polyol such as polypropylene glycol being a reactive substrate. Its solubility is not sufficiently high also in a low molecular weight diol such as ethylene glycol, propylene glycol or diethylene glycol, or in an aprotic solvent such as acetone, methyl ethyl ketone or ethyl acetate, and thus, there has been a problem such that when it is diluted with such a solvent, the active ingredient concentration tends to be low.

Thus, the conventional catalyst composition using an iron compound has low stability to hydrolysis and has had a problem in the stability in the coexistence of water. Therefore, when it is applied to a water blown foam, its activity is low, and it has been difficult to store it for a long period of time in a polyol premix containing water. Further, also in a non-water blown application where no water is added, there has been a problem that the reactivity decreases as time passes due to the moisture remaining in the urethane raw material.

### PRIOR ART DOCUMENTS

### PATENT DOCUMENTS

Patent Document 1: U.S. Patent No. 2,933,462
Patent Document 2: U.S. Patent No. 4,598,136
Patent Document 3: U.S. Patent No. 5,733,945

JP-A-2011 219605 discloses a catalyst composition comprising a compound of formula 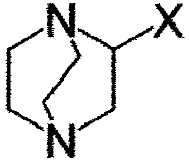 wherein X represents a hydroxy group, a hydroxymethyl group or a hydroxyethyl group, wherein said catalyst composition may optionally comprise other catalysts, with a list of examples as follows : organometallic catalysts, carboxylic acid metal salt catalysts, tertiary amine catalysts, quaternary ammonium salt catalysts, and the like.

This document also discloses examples of organometallic catalysts, carboxylic acid metal salt, tertiary amine catalysts, and quaternary ammonium salt catalysts.

As examples of organometallic catalysts, a number of compounds are cited, including iron acetylacetonate, zirconium acetylacetonate, nickel acetylacetonate, and cobalt acetylacetonate,
Examples 1-4 and Comparative Example 1 of this document discloses a composition catalyst comprising 2-hydroxymethyl triethylenediamine and N,N,N'-trimethyl-N'-(2-hydroxyethyl)bis(2-aminoethyl)ether.

### DISCLOSURE OF INVENTION

### TECHNICAL PROBLEM

The present invention has been made in view of the above-described background art, and its object is to provide a catalyst composition for polyurethane resin production, which has high gelling reactivity even in the coexistence of water, which is stable enough to be stored for a long period of time in a polyol premix containing water and which does not contain a harmful metal compound such as a tin or lead compound, and to provide a method for producing a polyurethane resin using such a catalyst composition.

### SOLUTION TO PROBLEM

As a result of an extensive study made in order to solve the above problem, the present inventors have found it possible to solve the problem by using a catalyst composition comprising a specific iron compound and a specific tertiary amine compound, and have accomplished the present invention.

That is, the present invention provides a catalyst composition for polyurethane resin production and a method for producing a polyurethane resin using it, as shown below.
[1] A catalyst composition for polyurethane resin production, which comprises an iron compound represented by the following formula (1) and a tertiary amine compound represented by the following formula (3):

   Feⁿ⁺(L)ₙ (1)

   [in the formula (1), each L independently is a β-diketonate ligand represented by the following formula (2), and n is 2 or 3], 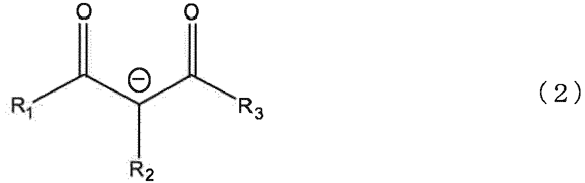 [in the formula (2), each of R₁ and R₃ which are independent of each other, is a C₂₋₁₀ alkyl group, and R₂ is a hydrogen atom or a C₁₋₇ alkyl group], 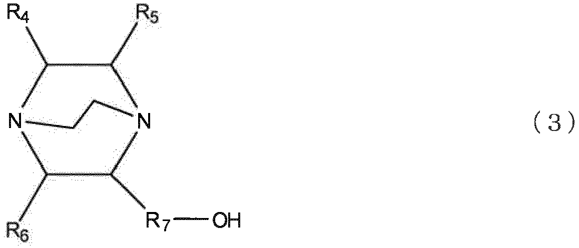 [in the formula (3), each of R₄ to R₆ which are independent of one another, is a hydrogen atom or a C₁₋₁₀ alkyl group, and R₇ is a C₁₋₁₀ alkylene group].
[2] The catalyst composition for polyurethane resin production according to the above [1], wherein in the β-diketonate ligand represented by the formula (2), and R₂ is a hydrogen atom.
[3] The catalyst composition for polyurethane resin production according to the above [1] or [2], wherein the β-diketonate ligand represented by the formula (2) is at least one member selected from the group consisting of 3,5-heptanedionate, 3,5-octanedionate, 2-methyl-3,5-heptanedionate, 2-methyl-3,5-nonanedionate and 2,6-dimethyl-3,5-heptanedionate.
[4] The catalyst composition for polyurethane resin production according to any one of the above [1] to [3], wherein in the tertiary amine compound represented by the formula (3), R₇ is a methylene group or an ethylene group.
[5] The catalyst composition for polyurethane resin production according to any one of the above [1] to [4], wherein the tertiary amine compound represented by the formula (3) is 2-hydroxymethyl-1 4-diazabicyclo[2.2.2]octane or 2-hydroxyethyl-1,4-diazabicyclo[2.2.2]octane.
[6] The catalyst composition for polyurethane resin production according to any one of the above [1] to [5], which contains at least 1 mol and at most 50 mol of the tertiary amine compound represented by the formula (3) relative to 1 mol of the iron compound represented by the formula (1).
[7] A method for producing a polyurethane resin, which comprises reacting a polyol component and an organic polyisocyanate component in the presence of the catalyst composition for polyurethane resin production as defined in any one of the above [1] to [6].
[8] A method for producing a polyurethane resin, which comprises reacting a polyol component and an organic polyisocyanate component in the presence of the catalyst composition for polyurethane resin production as defined in any one of the above [1] to [6] and a blowing agent.
[9] A method for producing a polyurethane resin, which comprises reacting a polyol premix comprising the catalyst composition for polyurethane resin production as defined in any one of the above [1] to [6], a polyol component and a blowing agent, with an organic polyisocyanate component.
[10] The method for producing a polyurethane resin according to the above [8] or [9], wherein the blowing agent contains water.

### ADVANTAGEOUS EFFECTS OF INVENTION

According to the present invention, it is possible to provide a catalyst composition for polyurethane resin production, which has high gelling reactivity even in the coexistence of water, which is stable enough to be stored for a long period of time in a polyol premix containing water and which does not contain a harmful metal compound such as a tin or lead compound, and to provide a method for producing a polyurethane resin using such a catalyst composition.

### DESCRIPTION OF EMBODIMENTS

Now, the present invention will be described in detail.

The catalyst composition for polyurethane resin production of the present invention is characterized by comprising an iron compound represented by the above formula (1) and a tertiary amine compound represented by the above formula (3).

In the above formula (1), each L independently is a β-diketonate ligand represented by the above formula (2).

In the formula (2), each of R₁ and R₃ which are independent of each other, is a C₂₋₁₀ alkyl group, and R₂ is a hydrogen atom or a C₁₋₇ alkyl group.

The C₁₋₁₀ alkyl group is not particularly limited, and, for example, a methyl group (Me), an ethyl group (Et), a n-propyl group (Pr), an isopropyl group (iPr), a cyclopropyl group, a n-butyl group (Bu), an isobutyl group (iBu), a s-butyl group, a t-butyl group, a cyclobutyl group, a n-pentyl group, an isopentyl group, a neopentyl group, a t-pentyl group, a cyclopentyl group, a n-hexyl group, a 1-methylpentyl group, a 4-methyl-2-pentyl group, a 3,3-dimethylbutyl group, a 2-ethylbutyl group, a cyclohexyl group, a n-heptyl group, a 1-methylhexyl group, a cycloheptyl group, a n-octyl group, a t-octyl group, a 1-methylheptyl group, a 2-ethylhexyl group, a 2-propylpentyl group, a cyclooctyl group, a n-nonyl group, a 2,2-dimethylheptyl group, a 2,6-dimethyl-4-heptyl group, a 3,5,5-trimethylhexyl group, a n-decyl group, may be mentioned.

The iron compound represented by the above formula (1) is not particularly limited, but, for example, as compounds with the above formula (2) not part of the invention wherein either R₁ or R₃ is a methyl group, and R₂ is a hydrogen atom or an alkyl group, specifically, iron tris(2,4-pentanedionate), iron tris(2,4-hexanedionate), iron tris(2,4-heptanedionate), iron tris(octanedionate), iron tris (5-methyl-2,4-hexanedionate), iron tris(6-methyl-2,4-heptanedionate), iron tris(3-methyl-2,4-pentanedionate), iron tris(3-ethyl-2,4-pentanedionate), iron tris(3-methyl-2,4-hexanedionate), iron tris(3-methyl-2,4-octanedionate), iron tris(3,6-dimethyl-heptanedionate), and the corresponding divalent iron compounds may be exemplified. These compounds are solid at ordinary temperatures, and therefore, they will be usually diluted with an organic solvent, when used for catalyst compositions.

As compounds with the above formula (2) wherein each of R₁ and R₃ is an alkyl group having at least 2 carbon atoms, and R₂ is a hydrogen atom or an alkyl group, specifically, iron tris(3,5-heptanedionate), iron tris(3,5-octanedionate), iron tris(2-methyl-3,5-heptanedionate), iron tris(3,5-nonanedionate), iron tris(4,6-nonanedionate), iron tris(2-methyl-3,5-octanedionate), iron tris(2,6-dimethyl-3,5-heptanedionate), iron tris(7-methyl-3,5-heptanedionate), iron tris(2,2-dimethyl-3,5-heptanedionate), iron tris(3,5-decanedionate), iron tris(4,6-decanedionate), iron tris(2-methyl-3,5-nonanedionate), iron tris(2,7-dimethyl-3,5-octanedionate), iron tris(2,2-dimethyl-3,5-octanedionate), iron tris(2,2,6-trimethyl-3,5-heptanedionate), iron tris(3,5-undecanedionate), iron tris(4,6-undecanedionate), iron tris(2-methyl-3,5-undecanedionate), iron tris(2-methyl-4,6-undecanedionate), iron tris(2,2-dimethyl-3,5-nonanedionate), iron tris(2,2,7-trimethyl-3,5-octanedionate), iron tris(2,2,6,6-tetramethyl-3,5-heptanedionate), iron tris(4-methyl-3,5-heptanedionate), iron tris(4-ethyl-3,5-octanedionate), and the corresponding divalent iron compounds may be exemplified.

These compounds have high water resistance by the iron compounds themselves as compared with the compounds wherein either R₁ or R₃ is a methyl group, and thus, they present catalyst compositions having high water resistance. Further, such compounds are liquid at ordinary temperatures or have high solubility in a diol compound, and thus, they are advantageous when used to prepare catalyst compositions.

Suitable as the β-diketonate ligand represented by the above formula (2) is, for example, 3,5-heptanedionate, 3,5-octanedionate, 2-methyl-3,5-heptanedionate, 2-methyl-3,5-nonanedionate, 2,6-dimethyl-3,5-heptanedionate.

Preferred as the iron compound represented by the formula (1) is, for example, iron tris(3,5-heptanedionate), iron tris(3,5-octanedionate), iron tris(2-methyl-3,5-heptanedionate), iron tris(2-methyl-3,5-octanedionate), iron tris(2-methyl-3,5-nonanedionate) or iron tris(2,7-dimethyl-3,5-octanedionate), since it is liquid at ordinary temperature and also has high water resistance.

The β-diketonate ligand represented by the above formula (2) is an anion of a β-diketone. The corresponding β-diketone may be synthesized, for example, as follows, although its synthetic method is not particularly limited. That is, a metal base such as sodium hydride, sodium ethoxide or potassium tertiary butoxide, is dissolved or dispersed in an aprotic solvent such as dimethylformamide, and a mixed liquid of the corresponding ester and ketone, is dropwise added and reacted at from 20 to 100°C for from 1 to 10 hours (Claisen condensation). After completion of the reaction, hydrochloric acid is added and then, the β-diketone is extracted with an organic solvent. The organic layer is concentrated and then distilled to obtain the desired β-diketone.

The iron compound represented by the formula (1) may be synthesized, for example, as follows, although its synthetic method is not particularly limited. That is, iron(III) chloride (anhydrous) or iron(III) chloride hexahydrate is dissolved in methanol, and then, a β-diketone is added in an amount of from 3 to 5 mol equivalents to iron atoms. After completion of the reaction, distilled water and then triethylamine are added to separate an iron compound. The separated iron compound is extracted with an organic solvent such as diethyl ether and then dried under reduced pressure to obtain the desired iron compound.

The tertiary amine compound to be used in the present invention is an alkanolamine compound having a triethylenediamine (hereinafter abbreviated as "TEDA") skeleton, represented by the above formula (3).

In the above formula (3), each of R₄ to R₆ which are independent of one another, is a hydrogen atom or a C₁₋₁₀ alkyl group, and R₇ is a C₁₋₁₀ alkylene group.

The C₁₋₁₀ alkyl group is not particularly limited, and, for example, a methyl group, an ethyl group, a n-propyl group, an isopropyl group, a cyclopropyl group, a n-butyl group, an isobutyl group, a s-butyl group, a t-butyl group, a cyclobutyl group, a n-pentyl group, an isopentyl group, a neopentyl group, a t-pentyl group, a cyclopentyl group, a n-hexyl group, a 1-methylpentyl group, a 4-methyl-2-pentyl group, a 3,3-dimethylbutyl group, a 2-ethylbutyl group, a cyclohexyl group, a n-heptyl group, a 1-methylhexyl group, a cycloheptyl group, a n-octyl group, a t-octyl group, a 1-methylheptyl group, a 2-ethylhexyl group, a 2-propylpentyl group, a cyclooctyl group, a n-nonyl group, a 2,2-dimethylheptyl group, a 2,6-dimethyl-4-heptyl group, a 3,5,5-trimethylhexyl group, a n-decyl group, may be mentioned.

The C₁₋₁₀ alkylene group is not particularly limited, and, for example, a methylene group, an ethylene group, a propylene group, a butylene group, a pentylene group, a hexylene group, a heptylene group, an octylene group, a nonylene group, a decylene group, may be mentioned.

The tertiary amine compound represented by the above formula (3) is not particularly limited, and specifically, 2-hydroxymethyl-1,4-diazabicyclo[2.2.2]octane (hydroxymethyl TEDA), 2-hydroxyethyl-1,4-diazabicyclo[2.2.2]octane, 2-hydroxypropyl-1,4-diazabicyclo[2.2.2]octane, 2-hydroxymethyl-3-methyl-1,4-diazabicyclo[2.2.2]octane, 2-hydroxymethyl-5-methyl-1,4-diazabicyclo[2.2.2]octane, 2-hydroxymethyl-6-methyl-1,4-diazabicyclo[2.2.2]octane, 2-hydroxymethyl-3-ethyl-1,4-diazabicyclo[2.2.2]octane, 2-hydroxymethyl-5-ethyl-1,4-diazabicyclo[2.2.2]octane, 2-hydroxymethyl-6-ethyl-1,4-diazabicyclo[2.2.2]octane, may be exemplified. Among these compounds, 2-hydroxymethyl-1,4-diazabicyclo[2.2.2]octane or 2-hydroxyethyl-1,4-diazabicyclo[2.2.2]octane is particularly suitably used from the viewpoint of the catalytic activities.

The method for producing the tertiary amine compound represented by the above formula (3) is not particularly limited, and, for example, a method of reducing an ester obtained by reacting piperazine with ethyl 2,3-dibromopropanoate (see e.g. JP-A-2001-504855), a method of subjecting a piperazine and glycerin to a dehydration condensation reaction in the presence of an acid catalyst (see e.g. JP-A-2010-105944), or a method of subjecting a dihydroxyalkylpiperazine derivative to an intramolecular dehydration condensation reaction in the presence of an acid catalyst (see e.g. JP-A-2011-037819) may be mentioned.

In the present invention, the mixing ratio of the iron compound represented by the above formula (1) and the tertiary amine compound represented by the above formula (3) is not particularly limited, and, for example, the mixing ratio is adjusted so that relative to 1 mol of the iron compound, the tertiary amine compound would be in a range of preferably at least 1 mol and at most 50 mol, more preferably at least 5 mol and at most 20 mol. If the tertiary amine compound is less than 1 mol relative to 1 mol of the iron compound, the change with time of the reactivity in the presence of water tends to be large, and the practical efficiency tends to be poor. On the other hand, if the tertiary amine compound exceeds 50 mol relative to 1 mol of the iron compound, the foaming activity tends to be too high, so that formation of a polyurethane foam is likely to be difficult.

The catalyst composition for polyurethane resin production of the present invention is not particularly required to contain components other than the above-described essential components. However, for the purpose of improving the uniformity of the composition, adjusting the reactivity and improving the water resistance, it may contain, for example, a β-diketone compound, a carboxylic acid, a phenol, an aprotic solvent, in addition to the above-described essential components.

The β-diketone compound includes, for example, 2,4-pentanedione (actylacetone), 2,4-hexanedione, 3,5-heptanedione, 2-methyl-3,5-hexanedione, 6-methyl-2,4-heptanedione, 2,6-dimethyl-3,5-heptanedione, 2,2-dimethyl-3,5-hexanedione, 2,2,6,6-tetramethyl-3,5-heptanedione, methyl acetoacetate, ethyl acetoacetate, isopropyl acetoacetate, tert-butyl acetoacetate, methyl propionylacetate, ethyl propionylacetate, isopropyl propionylacetate, tert-butyl propionylacetate, methyl isobutylylacetate, ethyl isobutylylacetate, isopropyl isobutylylacetate, tert-butyl isobutylylacetate, methyl 4,4-dimethyl-3-oxopentanoate, ethyl 4,4-dimethyl-3-oxopentanoate, isopropyl 4,4-dimethyl-3-oxopentanoate, tert-butyl 4,4-dimethyl-3-oxopentanoate.

The carboxylic acid includes, for example, formic acid, acetic acid, 2-ketoethanoic acid (glyoxylic acid), propanoic acid, 2-ketopropanoic acid (pyruvic acid), butanoic acid, isobutanoic acid, 2-ketobutanoic acid, pentanoic acid, isopentanoic acid, hexanoic acid, 2-methylpentanoic acid, 2-ethylbutanoic acid, heptanoic acid, caprylic acid, 2-ethylhexanoic acid, nonanoic acid, undecylic acid, lauric acid, myristic acid, palmitic acid, stearic acid.

Further, the carboxylic acid includes a hydroxycarboxylic acid having a hydroxy group. For example, 2-hydroxyethanoic acid (glycolic acid), 2-phenyl-2-hydroxyethanoic acid (mandelic acid), 2-hydroxypropanoic acid (lactic acid), 3-hydroxypropanoic acid, 2-hydroxy-1,2,3-propanetricarboxylic acid (citric acid), 1-hydroxy-1,2,3-propanetricarboxylic acid (isocitric acid), 2-hydroxybutanoic acid, 3-hydroxybutanoic acid, 2,2-bis(hydroxymethyl)butanoic acid, 2-hydroxybutane-1,4-diacid (malic acid), 2,3-dihydroxybutane-1,4-diacid (tartaric acid), 2-hydroxy-2-methylbutane-1,4-diacid (citramalic acid), salicylic acid, 3-methylsalicylic acid, 4-methylsalicylic acid, 5-methylsalicylic acid, 3-methoxysalicylic acid, 4-methoxysalicylic acid, 5-methoxysalicylic acid, may be mentioned.

The phenol includes, for example, phenol, 2-methylphenol, 3-methylphenol, 4-methylphenol, 2,3-dimethylphenol, 3,4-dimethylphenol, 3,5-dimethylphenol, 2,6-dimethylphenol, 2-methoxyphenol, 3-methoxyphenol, 4-methoxyphenol, 2-acetylphenol, 3-acetylphenol, 4-acetylphenol, 1-naphthol, 2-naphthol, 2-methoxycarbonylphenol, 2-ethoxycarbonylphenol.

The alcohol includes, for example, methanol, ethanol, 1-propanol, 2-propanol, 1-butanol, 1-hexanol, 1-octanol, 2-ethylhexanol, ethylene glycol, propylene glycol, diethylene glycol, dipropylene glycol, 1,4-butanediol, 1, 6-hexanediol. Among these alcohols, ethylene glycol, diethylene glycol, dipropylene glycol or 1,4-butanediol is preferred from the viewpoint of the catalytic activities.

The aprotic solvent includes, for example, acetone, methyl ethyl ketone, ethyl acetate, butyl acetate, tetrahydrofuran, acetonitrile, dimethylformamide, dimethylacetamide. Among these aprotic solvents, methyl ethyl ketone, dimethylformamide or dimethylacetamide is preferred from the viewpoint of the catalytic activities.

Further, the catalyst composition of the present invention contains preferably from 3 to 50 wt%, more preferably from 5 to 30 wt%, of the iron compound represented by the above formula (1).

The catalyst composition of the present invention may be used without any particular restrictions so long as the essential components are mixed at the time of use as a catalyst. The mixing form and the method of use of the catalyst composition may, for example, be
(a) a method wherein a catalyst composition comprising an iron compound represented by the above formula (1) and a tertiary amine compound represented by the above formula (3) is preliminarily prepared and added to a polyol component,
(b) a method wherein a catalyst composition comprising an iron compound represented by the above formula (1) and a tertiary amine compound represented by the above formula (3) is preliminarily mixed and added to an organic polyisocyanate component, or
(c) a method wherein an iron compound represented by the above formula (1) is added to an organic polyisocyanate component, and a tertiary amine compound represented by the above formula (3) is added to a polyol component, respectively.

The catalyst composition of the present invention is useful for the method for producing a foamed or non-foamed polyurethane resin.

The method for producing a polyurethane resin of the present invention is characterized by comprising reacting a polyol component and an organic polyisocyanate component in the presence of the above-described catalyst composition for polyurethane resin production of the present invention.

Further, in the production method of the present invention, in a case where a polyol component and a polyisocyanate component are reacted in the presence of the above-described catalyst composition for polyurethane resin production of the present invention and a blowing agent, a foamed polyurethane resin (polyurethane foam) will be obtained. At that time, a polyol premix comprising the above-described catalyst composition for polyurethane resin production of the present invention, a polyol component and a blowing agent may be prepared, and such a premix may be reacted with an organic polyisocyanate component.

In the production method of the present invention, the amount of the catalyst composition of the present invention to be used, is such that when the polyol component to be used is taken as 100 parts by weight, the weight of the iron compound represented by the above formula (1) contained in the catalyst composition is usually within a range of from 0.001 to 10 parts by weight, preferably within a range of from 0.01 to 1 part by weight.

In the production method of the present invention, the polyol to be used as the polyol component is not particularly limited, and, for example, a conventional polyether polyol, polyester polyol, polymer polyol, vegetable oil polyol, and further, a flame-retardant polyol such as a phosphorus-containing polyol or halogen-containing polyol, may be used. These polyols may be used alone, or may be used in combination as suitably mixed.

The polyether polyol is not particularly limited and may, for example, be one produced by an addition reaction of a compound having at least two active hydrogen groups (specifically a polyhydric alcohol such as ethylene glycol, propylene glycol, glycerin, trimethylolpropane or pentaerythritol, an amine such as ethylenediamine, or an alkanolamine such as ethanolamine or diethanolamine is exemplified) as a starting material, with an alkylene oxide (specifically ethylene oxide or propylene oxide is exemplified) [see e.g. the method disclosed by Gunter Oertel, in "Polyurethane Handbook" (1985), Hanser Publishers (Germany), p. 42-53].

The polyester polyol is not particularly limited and may, for example, be a condensation reaction product of a polyhydric carboxylic acid such as adipic acid or phthalic acid with a polyhydric alcohol such as ethylene glycol, 1,4-butanediol or 1,6-hexanediol, or a polyester polyol obtained by treating a waste product such as a waste from nylon production, a waste of trimethylolpropane and pentaerythritol or a waste of a phthalic acid type polyester [see e.g. the disclosure by Keiji Iwata, in "Polyurethane Resin Handbook", (1987), Nikkan Gogyo Shimbun Ltd., p. 117].

The polymer polyol is not particularly limited and may, for example, be a polymer polyol obtained by reacting the above-mentioned polyether polyol with an ethylenically unsaturated monomer (e.g. butadiene, acrylonitrile or styrene may be mentioned) in the presence of a radical polymerization catalyst. As such a polymer polyol, one having a molecular weight of from about 5,000 to 12,000 is particularly preferred.

The vegetable oil polyol is not particularly limited and may, for example, be a hydroxy group-containing vegetable oil such as castor oil or coconut oil. Further, a castor oil derivative polyol obtained by using castor oil or hydrogenated castor oil as raw material may also be suitably used. The castor oil derivative polyol may, for example, be a castor oil polyester obtainable by a reaction of castor oil, a polybasic carboxylic acid and a short chain diol, or an alkylene oxide adduct of castor oil or castor oil polyester.

The flame-retardant polyol is not particularly limited and may, for example, be a phosphorus-containing polyol obtainable by adding an alkylene oxide to a phosphoric acid compound, a halogen-containing polyol obtainable by subjecting epichlorohydrin or trichlorobutylene oxide to ring-opening polymerization, an aromatic ether polyol obtainable by adding an alkylene oxide to an active hydrogen compound having an aromatic ring, or an aromatic ester polyol obtainable by a condensation reaction of a polybasic carboxylic acid having an aromatic ring with a polyhydric alcohol.

The hydroxy value of the above polyol is preferably from 5 to 300 mgKOH/g, more preferably from 10 to 250 mgKOH/g. The hydroxy value may be measured by a method stipulated in JIS-K0070.

In the production method of the present invention, the organic polyisocyanate to be used as the organic isocyanate component is not particularly limited, and, for example, not only a conventional polyisocyanate monomer, but also its polymeric derivative may be used. The polyisocyanate monomer may, for example, be an aromatic polyisocyanate such as toluene diisocyanate (hereinafter sometimes referred to as "TDI"), 4,4'-diphenylmethane diisocyanate (hereinafter sometimes referred to as "MDI"), 4,4'-diphenyl ether diisocyanate, naphthalene diisocyanate, xylene-1,3-diisocyanate, xylene-1,4-diisocyanate, 2-nitrodiphenyl-4,4'-diisocyanate or xylylene diisocyanate, an aliphatic polyisocyanate such as hexamethylene diisocyanate, an alicyclic polyisocyanate such as dicyclohexyl diisocyanate or isophorone diisocyanate, or a mixture thereof. As TDI and its derivative, a mixture of 2,4-toluene diisocyanate and 2,6-toluene diisocyanate, or a terminal isocyanate prepolymer of TDI, may, for example, be mentioned. Further, as MDI and its derivative, a mixture of MDI and a polyphenyl-polymethylene diisocyanate of its polymer, and/or a diphenylmethane diisocyanate derivative having terminal isocyanate groups may, for example, be mentioned.

In the production method of the present invention, a urethane prepolymer may also be used as the organic polyisocyanate component. The urethane prepolymer may be produced, for example, by reacting the above-mentioned polyol with an organic polyisocyanate monomer. At that time, the molar ratio of isocyanate groups to hydroxy groups is set preferably within a range of from 1 to 3.5, and the reaction temperature is adjusted preferably within a range of from 0 to 150°C.

In the production method of the present invention, the isocyanate index is not particularly limited and is usually within a range of from 70 to 250. The isocyanate index is defined by the total amount of isocyanate groups/the total amount of hydroxy groups (molar ratio) × 100.

In the production method of the present invention, in addition to the catalyst composition of the present invention, other catalyst may be used in combination. As such other catalyst, a conventional metal compound, tertiary amine compound or quaternary ammonium salt compound may, for example, be mentioned.

The metal compound may be a conventional one and is not particularly limited. For example, stannous diacetate, stannous dioctoate, stannous dioleate, stannous dilaurate, dibutyltin oxide, dibutyltin diacetate, dibutyltin dilaurate, dibutyltin dichloride, dioctyltin dilaurate, lead octanoate, lead naphthenate, nickel naphthenate, cobalt naphthenate, may be mentioned.

The tertiary amine compound may be a conventional one and is not particularly limited. For example, tertiary amine compounds such as N,N,N',N'-tetramethylethylenediamine, N,N,N',N'-tetramethylpropylenediamine, N,N,N',N",N"-pentamethyldiethylenetriamine, N,N,N',N",N"-pentamethyl-(3-aminopropyl)ethylenediamine, N,N,N',N",N"-pentamethyldipropylenetriamine, N,N,N',N'-tetramethylguanidine, 1,3,5-tris(N,N-dimethylaminopropyl)hexahydro-S-triazine, 1,8-diazabicyclo[5.4.0]undecene-7, triethylenediamine, N,N,N',N'-tetramethylhexamethylenediamine, N-methyl-N'-(2-dimethylaminoethyl)piperazine, N,N'-dimethylpiperazine, dimethylcyclohexylamine, N-methylmorpholine, N-ethylmorpholine, bis(2-dimethylaminoethyl) ether, 1-methylimidazole, 1,2-dimethylimidazole, 1-isobutyl-2-methylimidazole, 1-dimethylaminopropylimidazole, may be mentioned.

The quaternary ammonium salt compound may be a conventional one and is not particularly limited. For example, a tetraalkylammonium halide such as tetramethylammonium chloride, a tetraalkylammonium hydroxide such as tetramethylammonium hydroxide, or a tetraalkylammonium organic acid salt such as tetramethylammonium 2-ethylhexanoate, 2-hydroxypropyltrimethylammonium formate or 2-hydroxypropyltrimethylammonium 2-ethylhexanoate, may be mentioned.

In the production method of the present invention, in the case of non-foaming procedure using no blowing agent, it is possible that a foaming phenomenon occurs due to moisture in the reaction system, and therefore, it is desirable to remove moisture. For the removal of moisture, it is preferred not only to carry out reduced pressure dehydration under heating with respect to raw materials such as a polyol, prepolymer, but also to add a molecular sieve, zeolite to the system. Further, if necessary, it is possible to use a defoaming agent.

Further, in the production method of the present invention, in the case of foaming procedure using a blowing agent, the blowing agent is, for example, preferably water, a hydrocarbon blowing agent or a halogenated hydrocarbon blowing agent.

The hydrocarbon blowing agent may, for example, be methane, ethane, propane, butane, isobutane, pentane, isopentane, cyclopentane, hexane.

The halogenated hydrocarbon blowing agent may, for example, be a halogenated methane, a halogenated ethane, a fluorinated hydrocarbon. Specifically, methylene chloride, HCFC-141b, HFC-245fa, HFC-356mfc may be exemplified.

In use of such a blowing agent, water, the hydrocarbon blowing agent and the halogenated blowing agent may be used alone, respectively, or they may be used in combination. The amount of the blowing agent varies depending upon the density of the desired polyurethane product and is not particularly limited, but it is usually at least 0.1 part by weight, preferably within a range of from 0.5 to 10.0 parts by weight per 100 parts by weight of the polyol component.

In the production method of the present invention, if necessary, it is possible to use a surfactant. The surfactant to be used in the production method of the present invention may, for example, be an organic silicone surfactant, but is not particularly limited thereto. Its amount is usually within a range of from 0.1 to 10 parts by weight per 100 parts by weight of the polyol component.

In the production method of the present invention, if necessary, it is possible to use a crosslinking agent or chain extender. As the crosslinking agent or chain extender, for example, a low molecular weight polyhydric alcohol, a low molecular weight amine polyol, a polyamine, may be mentioned.

The low molecular weight polyhydric alcohol may, for example, be ethylene glycol, 1,4-butanediol or glycerin.

The low molecular weight amine polyol may, for example, be diethanolamine or triethanolamine.

The polyamine may, for example, be ethylenediamine, diethylenetriamine, triethylenetetramine, hexamethylenepentamine, bisaminopropylpiperazine, tris(2-aminoethyl)amine, isophoronediamine or a polyoxyalkylenepolyamine.

In the production method of the present invention, among these crosslinking agents or chain extenders, diethanolamine or triethanolamine is preferred.

In the production method of the present invention, as the case requires, it is possible to further use a colorant, a flame retardant, an anti-aging agent, a filler, a thickener, a plasticizer, an UV absorber, a solvent, a thixotropic agent, other known additives. The types and amounts of these additives may preferably be within the respective ranges not to depart from the conventional types and procedures.

### EXAMPLES

Now, the present invention will be described with reference to Preparation Example, Working Examples, Comparative Examples and Evaluation Examples, but it should be understood that the present invention is by no means limited to such Examples. Compounds prepared in Examples were analyzed under the following conditions.

### (Conditions for GC analysis)

Apparatus: GC-2014 (manufactured by Shimadzu Corporation), column: DB-5 (inner diameter 0.32 mm, length 30 m, membrane thickness 0.25 µm) (manufactured by GL Sciences), carrier: helium, split ratio: 40, injector temperature: 280°C, detection method: FID, injection amount: 1 µL, temperature program: raising from 50°C to 200°C at a rate of 10°C /min.

### (Elemental analysis)

A solid sample was dissolved, and the metal concentration was analyzed by ICP-AES method. Apparatus: OPTIMA3000DV (manufactured by Perkin Elmer Co., Ltd.).

Raw materials, etc. used in Examples and Comparative Examples are specifically ones shown below.

### (Raw materials for metal compounds)

2,4-Pentanedione: reagent manufactured by Tokyo Chemical Industry Co., Ltd.
3,5-Heptanedione: reagent manufactured by Sigma Aldrich Co.
6-Methyl-2,4-heptanedione: reagent manufactured by Tokyo Chemical Industry Co., Ltd.
2,6-Dimethyl-3,5-heptanedione: reagent manufactured by Tokyo Chemical Industry Co., Ltd.
2-Methyl-3,5-nonanedione and other β-ketones: prepared products
Iron(III) chloride (anhydrous): reagent manufactured by Kishida Chemical Co., Ltd.

### (Raw materials for polyurethanes)

Polyoxypropylene triol: number average molecular weight 3000, hydroxy value 56.5 mg/KOH/g (manufactured by Sanyo Chemical Industries, Ltd., tradename "Sanix GP3000")
Polyether polyol: hydroxy value 32.5 mg/KOH/g (manufactured by Sanyo Chemical Industries, Ltd., tradename "FA-703")
Polymer polyol: hydroxy value 20.5 mg/KOH/g (manufactured by Sanyo Chemical Industries, Ltd., tradename "KC-900")
MDI: diphenylmethane diisocyanate (manufactured by Nippon Polyurethane Industry Co., Ltd, tradename "MR-200")
TDI: tolylene diisocyanate (manufactured by Nippon Polyurethane Industry Co., Ltd, tradename "T80")

### PREPARATION EXAMPLE <Preparation of 2-methyl-3,5-nonanedione and other β-ketones>

Into a flask having a nitrogen atmosphere, 67.3g (0.6 mol) of potassium tertiary butoxide and 100.0 g of dimethylformamide were added, followed by heating at 50°C for dissolution. To this solution, a mixed liquid of 116.2 g (1.0 mol) of methyl valerate and 43.1 g (0.5 mol) of 3-methyl-2-butanone, was dropwise added over a period of two hours. After completion of the dropwise addition, the reaction was continued for further 5 hours.

To the total amount of the reaction liquid, 100.0 g of distilled water and then, 28.0 g of 12N hydrochloric acid were added. When the liquid temperature returned to room temperature, 50.0 g of diethyl ether was added, and the organic layer was recovered.

The organic layer was concentrated and then distilled under reduced pressure to obtain a slightly yellow liquid. This liquid was analyzed by GC and was found to contain 52.8 g of 2-methyl-3,5-nonanedione. The yield based on 3-methyl-2-butanone was 62%.

Other β-ketones (2,4-hexanedione, 5-methyl-2,4-hexanedione, 3,5-octanedione, 2-methyl-3,5-heptanedione, 4-methyl-3,5-heptanedione and 4-ethyl-3,5-octanedione) were prepared in the same manner as above using ester and ether raw materials shown in Table 1.

The preparation results or manufacturers of β-ketones are summarized in Table 1.

**[Table 1]**

| β-diketones (*1) | | | | | Preparation results (or manufacturers) | | |
|---|---|---|---|---|---|---|---|
| Compound name | Abbreviations (*2) | R₁ | R₂ | R₃ | Ester | Ketone | Yield (%) |
| 2,4-Pentanedione | AA-H | Me | H | Me | Manufactured by Tokyo Chemical Industry Co., Ltd. | | |
| 2,4-Hexanedione | HXDO-H | Et | H | Me | Methyl propionate | Acetone | 52 |
| 5-Methyl-2,4-hexanedione | 5-Me-HXDO-H | iPr | H | Me | Methyl isobutyrate | Acetone | 54 |
| 6-Methyl-2,4-heptanedione | 6-Me-HDO-H | iBu | H | Me | Manufactured by Tokyo Chemical Industry Co., Ltd. | | |
| 3,5-Heptanedione | HDO-H | Et | H | Et | Manufactured by Aldrich | | |
| 3,5-Octanedione | ODO-H | Pr | H | Et | Methyl butyrate | 2-Butanone | 55 |
| 2-Methyl-3,5-heptanedione | 2-Me-HDO-H | iPr | H | Et | Methyl propionate | 3-Methyl-2-butanone | 58 |
| 2,6-Dimethyl-3,5-heptanedione | 2,6-Me-HDO-H | iPr | H | iPr | Manufactured by Tokyo Chemical Industry Co., Ltd. | | |
| 2-Methyl-3,5-nonanedione | 2-Me-NDO-H | Bu | H | iPr | Methyl valerate | 3-Methyl-2-butanone | 62 |
| 4-Methyl-3,5-heptanedione | 4-Me-HDO-H | Et | Me | Et | Methyl propionate | 3-Pentanone | 60 |
| 4-Ethyl-3,5-octanedione | 4-Et-ODO-H | Pr | Et | Et | Methyl propionate | 4-Heptanone | 59 |

| | | | | | | | |
|---|---|---|---|---|---|---|---|
| 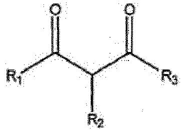 (*1) Structure of β-diketones: (*2) The corresponding anions are, respectively, represented by deleting the terminal (-H). | | | | | | | |

### EXAMPLE 1 <Preparation of catalyst 1A>

10.0 g of iron chloride (anhydrous) was dispersed in 100.0 g of methanol. To this solution, 25.3 g of 3,5-heptanedione was added to obtain a uniform solution. To this solution, 200.0 g of water was added, and then, 20.0 g of triethylamine was gradually added, whereby a red-colored oily substance was precipitated. The oily substance was extracted with 100.0 ml of diethyl ether, and the solvent was distilled off by a rotary evaporator, followed by drying under reduced pressure at 80°C to obtain 22.9 g of iron tris(3,5-heptanedionate) as a deep red-colored liquid. By the ICP method, the amount of iron element contained, was analyzed and was found to be 12.7 wt%, which agreed to the desired product.

Using the obtained iron tris(3,5-heptanedionate), the respective components were mixed in the proportions as shown in Table 2, to prepare catalyst 1A.

**[Table 2]**

| | Example 1 | Example 2 | Example 3 | Example 4 |
|---|---|---|---|---|
| Composition name | Catalyst 1A | Catalyst 2A | Catalyst 3A | Catalyst 4A |
| Iron compound | Fe(HDO)₃ | Fe(HDO)₃ | Fe(AA)₂(HDO) | Fe(AA)(HDO)₂ |
| R₁, R₂, R₃ | Et, H, Et | Et, H, Et | Et, H, Et | Et, H, Et |
| | | | Me, H, Me | Me, H, Me |
| Weight (g) | 0.50 | 0.50 | 0.50 | 0.50 |
| Tertiary amine compound | TEDA-OH | TEDA-OH | TEDA-OH | TEDA-OH |
| Weight (g) | 0.81 | 1.63 | 1.86 | 1.74 |
| Diol | Diethylene glycol | Diethylene glycol | Diethylene glycol | Diethylene glycol |
| Weight (g) | 0.33 | 0.53 | 1.01 | 0.96 |
| Tertiary amine compound/Iron compound Molar ratio (mol/mol) | 5 | 10 | 10 | 10 |
| Content of iron compound in composition (wt%) | 30.5 | 18.8 | 14.8 | 15.6 |

In Table 2, "TEDA-OH" is 2-hydroxymethyl-1,4-diazabicyclo[2.2.2]octane, and the same applies in the following Tables.

### EXAMPLE 2 <Preparation of catalyst 2A>

In the same manner as in Example 1, the respective components were mixed in the proportions as shown in Table 2 to prepare catalyst 2A.

### EXAMPLE 3 <Preparation of catalyst 3A> not forming part of the invention

10.0 g of iron chloride (anhydrous) was dispersed in 100.0 g of methanol. To this solution, 7.9 g of 3,5-heptanedione and then, 13.6 g of 2,4-pentandione were added to obtain a uniform solution. To this solution, 200.0 g of water was added, and then, 20.0 g of triethylamine was gradually added, whereby a red-colored oily substance was precipitated. The oily substance was extracted with 100.0 ml of diethyl ether, and the solvent was distilled off by a rotary evaporator, followed by drying under reduced pressure at 80°C to obtain 19.7 g of iron(III) bis(2,4-pentanedionate)(3,5-heptanedionate) as a deep red-colored solid. By the ICP method, the amount of iron element contained, was analyzed and was found to be 14.6 wt%, which agreed to the desired product as the average composition.

Using the obtained iron(III) bis(2,4-pentanedionate)(3,5-heptanedionate), the respective components were mixed in the proportions as shown in Table 2, to prepare catalyst 3A.

### EXAMPLE 4 <Preparation of catalyst 4A> not forming part of the invention

Iron(III) bis(3,5-heptanedionate)(2,4-pentanedionate) was prepared in the same manner as in Example 3 except that the ratio of 3,5-heptanedione and 2,4-pentandione was changed. Using the obtained iron(III) bis(3,5-heptanedionate)(2,4-pentanedionate), in the same manner as in Example 1, the respective components were mixed in the proportions as shown in Table 2, to prepare catalyst 4A. EXAMPLES 5 and 6 <Preparation of catalysts 5A and 6A> not forming part of the invention

In the same manner as in Example 1, the respective components were mixed in the proportions as shown in Table 3, to prepare catalysts 5A and 6A. In these Examples, the compositions were finally in the form of a slurry and were not uniformly dissolved, but in the subsequent evaluations, they were used as they were.

**[Table 3]**

| | Example 5 | Example 6 | Example 7 | Example 8 |
|---|---|---|---|---|
| Composition name | Catalyst 5A | Catalyst 6A | Catalyst 7A | Catalyst 8A |
| Iron compound | Fe(AA)₃ | Fe(AA)₂ | Fe(HDO)₂ | Fe(HDO)₂ |
| R₁, R₂, R₃ | Me, H, Me | Me, H, Me | Et, H, Et | Et, H, Et |
| Weight (g) | 0.50 | 0.50 | 0.50 | 0.50 |
| Tertiary amine compound | TEDA-OH | TEDA-OH | TEDA-OH | TEDA-OH |
| Weight (g) | 2.01 | 2.80 | 1.15 | 2.29 |
| Diol | Diethylene glycol | Diethylene glycol | Diethylene glycol | Diethylene glycol |
| Weight (g) | 1.08 | 1.41 | 0.41 | 0.70 |
| Tertiary amine compound/Iron compound Molar ratio (mol/mol) | 10 | 10 | 5 | 10 |
| Content of iron compound in composition (wt%) | 13.9 | 10.6 | 24.3 | 14.3 |

### EXAMPLE 7 <Preparation of catalyst 7A>

10.0 g of iron(II) sulfate heptahydrate was dispersed in methanol. To this solution, 10.1 g of 3,5-heptanedione was added to obtain a uniform solution. To this solution, 200.0 g of water was added, and then, 20.0 g of triethylamine was gradually added, whereby a red-colored oily substance was precipitated. The oily substance was extracted with 100.0 ml of diethyl ether, and the solvent was distilled off by a rotary evaporator, followed by drying under reduced pressure at 80°C to obtain 9.3 g of iron(II) bis(3,5-heptanedionate) as a deep red-colored liquid. By the ICP method, the amount of iron element contained, was analyzed and was found to be 17.9 wt%, which agreed to the desired product.

Using the obtained iron(II) bis(3,5-heptanedionate), the respective components were mixed in the proportions as shown in Table 3, to prepare catalyst 7A.

### EXAMPLE 8 <Preparation of catalyst 8A>

In the same manner as in Example 7, the respective components were mixed in the proportions as shown in Table 3 to prepare catalyst 8A.

### EXAMPLES 9 to 14 <Preparation of catalysts 9A to 14A>

A trisdiketonate iron compound was prepared in the same manner as in Example 1 except that the β-diketone was changed, and the respective components were mixed in the proportions as shown in Table 4 or 5, to prepare catalysts 9A to 14A.

**[Table 4]**

| | Example 9 | Example 10 | Example 11 | Example 12 |
|---|---|---|---|---|
| Composition name | Catalyst 9A | Catalyst 10A | Catalyst 11A | Catalyst 12A |
| Iron compound | Fe(ODO)₃ | Fe(2-Me-HDO)₃ | Fe(2-Me-NDO)₃ | Fe(2,6-Me-HDO)₃ |
| R₁, R₂, R₃ | Pr, H, Et | iPr, H, Et | Bu, H, iPr | iPr, H, iPr |
| Weight (g) | 0.50 | 0.50 | 0.50 | 0.50 |
| Tertiary amine compound | TEDA-OH | TEDA-OH | TEDA-OH | TEDA-OH |
| Weight (g) | 1.48 | 1.48 | 1.26 | 1.36 |
| Diol | Dipropylene glycol | Dipropylene glycol | Dipropylene glycol | Dipropylene glycol |
| Weight (g) | 0.85 | 0.85 | 0.75 | 0.80 |
| Tertiary amine compound/Iron compound Molar ratio (mol/mol) | 10.0 | 10.0 | 10.0 | 10.0 |
| Content of iron compound in composition (wt%) | 17.7 | 17.7 | 19.9 | 18.8 |

**[Table 5]**

| | Example 13 | Example 14 |
|---|---|---|
| Composition name | Catalyst 13A | Catalyst 14A |
| Iron compound | Fe(4-Me-HDO)₃ | Fe(4-Et-ODO)₃ |
| R₁, R₂, R₃ | Et, Me, Et | Pr, Et, Et |
| Weight (g) | 0.50 | 0.50 |
| Tertiary amine compound | TEDA-OH | TEDA-OH |
| Weight (g) | 1.48 | 1.26 |
| Diol | Dipropylene glycol | Dipropylene glycol |
| Weight (g) | 0.85 | 0.75 |
| Tertiary amine compound/Iron compound Molar ratio (mol/mol) | 10.0 | 10.0 |
| Content of iron compound in composition (wt%) | 17.6 | 19.9 |

### COMPARATIVE EXAMPLES 1 to 16 <Preparation of catalysts 1B to 16B>

In the same manner as in Example 1, the respective components were mixed in the proportions as shown in Tables 6 to 9, to prepare catalysts 1B to 16B. In Comparative Example 10 (catalyst 10B) and Comparative Example 16 (catalyst 16B) wherein iron tris(2,4-pentanedionate) was used as the iron compound, the compositions 24 were finally in the form of a slurry and were not uniformly dissolved, but in the subsequent evaluations, they were used as they were.

**[Table 6]**

| | Comparative Example 1 | Comparative Example 2 | Comparative Example 3 | Comparative Example 4 |
|---|---|---|---|---|
| Composition name | Catalyst 1B | Catalyst 2B | Catalyst 3B | Catalyst 4B |
| Iron compound | Fe(HDO)₃ | Fe(HDO)₃ | Fe(HDO)₃ | Fe(HDO)₃ |
| R₁, R₂, R₃ | Et, H, Et | Et, H, Et | Et, H, Et | Et, H, Et |
| Weight (g) | 0.50 | 0.50 | 0.50 | 0.50 |
| Alkanolamine | 2-Dimethylaminoethanol | 3-Dimethylamino-1-propanol | 1-Dimethylamino-2-propanol | 1-Hydroxymethyl-2-methylimidazole |
| Weight (g) | 1.02 | 1.18 | 1.18 | 1.28 |
| Diol | Nil | Nil | Nil | Diethylene glycol |
| Weight (g) | | | | 0.45 |
| Alkanolamine/Iron compound Molar ratio (mol/mol) | 10.0 | 10.0 | 10.0 | 10.0 |
| Content of iron compound in composition (wt%) | 32.9 | 29.8 | 29.8 | 22.4 |

**[Table 7]**

| | Comparative Example 5 | Comparative Example 6 | Comparative Example 7 | Comparative Example 8 |
|---|---|---|---|---|
| Composition name | Catalyst 5B | Catalyst 6B | Catalyst 7B | Catalyst 8B |
| Iron compound | Fe(HDO)₃ | Fe(HDO)₃ | Fe(HDO)₃ | Fe(HDO)₃ |
| R₁, R₂, R₃ | Et, H, Et | Et, H, Et | Et, H, Et | Et, H, Et |
| Weight (g) | 0.50 | 0.50 | 0.50 | 0.50 |
| Alkanolamine | 4(2-Hydroxyethyl)-morpholine | N,N,N'-trimethylamino-ethylethanolamine | 3-Quinuclidinol | Triethanolamine |
| Weight (g) | 1.50 | 1.67 | 1.45 | 1.36 |
| Diol | Nil | Nil | Diethylene glycol | Nil |
| Weiqht (g) | | | 0.84 | |
| Alkanolamine/Iron compound Molar ratio mol/mol | 10.0 | 10.0 | 10.0 | 10.0 |
| Content of iron compound in composition (wt%) | 25.0 | 23.0 | 17.9 | 26.8 |

**[Table 8]**

| | Comparative Example 9 | Comparative Example 10 | Comparative Example 11 | Comparative Example 12 |
|---|---|---|---|---|
| Composition name | Catalyst 9B | Catalyst 10B | Catalyst 11B | Catalyst 12B |
| Iron compound | Fe(HDO)₃ | Fe(AA)₃ | Fe(ODO)₃ | Fe(ODO)₃ |
| R₁, R₂, R₃ | Et, H, Et | Me, H, Me | Pr, H, Et | Pr, H, Et |
| Weight (g) | 0.50 | 0.50 | 0.50 | 0.50 |
| Tertiary amine | N,N-diethylhydroxylamine | 2-Dimethylaminoethanol | N,N,N'-trimethylamino-ethylethanolamine | 2-Dimethylaminoethanol |
| Weight (g) | 1.02 | 1.26 | 1.53 | 0.93 |
| Diol | Nil | Diethylene glycol | Nil | Nil |
| Weight (g) | | 0.76 | | |
| Tertiary amine compound/Iron compound Molar ratio (mol/mol) | 10.0 | 10.0 | 10.0 | 10.0 |
| Content of iron compound in composition (wt%) | 32.9 | 19.9 | 24.7 | 35.0 |

**[Table 9]**

| | Comparative Example 13 | Comparative Example 14 | Comparative Example 15 | Comparative Example 16 |
|---|---|---|---|---|
| Composition name | Catalyst 13B | Catalyst 14B | Catalyst 15B | Catalyst 16B |
| Iron compound | Fe(HDO)₃ | Fe(2-Me-NDO)₃ | Fe(HDO)₃ | Fe(AA)₃ |
| R₁, R₂, R₃ | Et, H, Et | Bu, H, iPr | Et, H, Et | Me, H, Me |
| Weight (g) | 0.50 | 0.50 | 0.50 | 0.50 |
| Tertiary amine | Nil | Nil | Triethylenediamine | Triethylenediamine |
| Weight (g) | | | 1.28 | 1.59 |
| Diol | Nil | Nil | Diethylene glycol | Diethylene glycol |
| Weight (g) | | | 0.76 | 0.89 |
| Alkanolamine/Iron compound Molar ratio (mol/mol) | - | - | 10.0 | 10.0 |
| Content of iron compound in composition (wt%) | 100.0 | 100.0 | 19.6 | 16.8 |

### EVALUATION EXAMPLE 1 <Evaluation of water resistance>

Polyoxypropylene triol (Sanix GP3000, manufactured by Sanyo Chemical Industries, Ltd.) was dried under reduced pressure at 130°C for 1 hour to prepare a dehydrated polyol. To 100.0 g of the dehydrated polyol, 164 mg of catalyst 1A prepared in Example 1 was dissolved (as the iron compound, 0.05 wt% to the polyol). Into a cup made of polyethylene, 10.0 g of the dehydrated polyol having the catalyst dissolved, and toluene diisocyanate (T80, manufactured by Nippon Polyurethane Industry Co., Ltd) in such an amount that the isocyanate index [isocyanate groups/OH groups (molar ratio) × 100] would be 105, were added and stirred for 15 seconds. A part of such a mixed liquid was permitted to quickly flow into a circular dent (diameter: 1 cm, depth: 2 mm) on a die preliminarily heated to 60°C, and the reactivity (gel time and tack-free time) was evaluated.

In order to evaluate the hydrolysis resistance (water resistance) of the catalyst, a similar test was carried out by using, instead of the above dehydrated polyol, a water-containing polyol prepared by adding 0.5 part by weight of deionized water to the dehydrated polyol. Further, in order to investigate the change with time of the reactivity, the water-containing polyol having the catalyst added, was stored for 7 days in an oven adjusted to 50°C, and a similar evaluation was again carried out. The evaluation results are shown in Table 10.

### [Measured items for reactivity]

Gel time: The time for change from a liquid state to a resinous state as the reaction proceeds.

Tack-free time: The time until tackiness felt by a finger disappears as the resin cures.

**[Table 10]**

| | Evaluation Example 1 | Evaluation Example 2 | Evaluation Example 3 | Evaluation Example 4 | Evaluation Example 5 |
|---|---|---|---|---|---|
| Corresponding Example/Comparative Example | Example 1 | Example 2 | Example 3 | Example 4 | Example 5 |
| Catalyst composition | Catalyst 1A | Catalyst 2A | Catalyst 3A | Catalyst 4A | Catalyst 5A |
| Weight (mg) | 164 | 266 | 338 | 320 | 359 |
| Dehydrated polyol | | | | | |
| Gel time (min) | 2.0 | 1.7 | 2.5 | 2.0 | 2.5 |
| Tack-free time (min) | 3.0 | 3.0 | 4.0 | 3.5 | 4.5 |
| Water-containing polyol: | | | | | |
| Immediate after preparation | | | | | |
| Gel time (min) | 3.5 | 3.0 | 5.5 | 4.3 | 6.5 |
| Tack-free time (min) | 5.5 | 5.0 | 9.0 | 7.5 | 11.0 |
| Water-containing polyol: | | | | | |
| Upon expiration of 7 days | | | | | |
| Gel time (min) | 5.0 | 3.5 | 8.0 | 6.5 | 10.0 |
| Tack-free time (min) | 7.5 | 5.5 | 12.5 | 10.0 | 15.5 |
| Storage stability | ○ | ○ | ○ | ○ | ○ |

| | | | | | |
|---|---|---|---|---|---|
| Storage stability "O": The storage stability is good. "×": The storage stability is no good. The same applies in the following Tables. | | | | | |

### EVALUATION EXAMPLES 2 to 14 <Evaluation of water resistance>

Evaluation Examples 2 to 14 (corresponding to Examples 2 to 14, respectively) were carried out in the same manner as in Evaluation Example 1 except that the catalyst composition and the amount added were changed as shown in Tables 10 to 12. The results are also shown in Tables 10 to 12. In such evaluations, the amount of the catalyst added, was 0.05 wt% to the polyol, as calculated as the iron compound.

Evaluation Examples 3, 4, 5, 6 use Catalysts 3A, 4A, 5A and 6A not forming part of the invention.

**[Table 11]**

| | Evaluation Example 6 | Evaluation Example 7 | Evaluation Example 8 | Evaluation Example 9 | Evaluation Example 10 |
|---|---|---|---|---|---|
| Corresponding Example/Comparative Example | Example 6 | Example 7 | Example 8 | Example 9 | Example 10 |
| Catalyst composition | Catalyst 6A | Catalyst 7A | Catalyst 8A | Catalyst 9A | Catalyst 10A |
| Weight (mg) | 472 | 350 | 206 | 283 | 283 |
| Dehydrated polyol | | | | | |
| Gel time (min) | 2.5 | 2.0 | 2.0 | 1.3 | 1.3 |
| Tack-free time (min) | 4.5 | 3.0 | 3.0 | 2.5 | 2.5 |
| Water-containing polyol: | | | | | |
| Immediate after preparation | | | | | |
| Gel time (min) | 7.0 | 3.5 | 4.0 | 3.5 | 4.0 |
| Tack-free time (min) | 11.5 | 5.5 | 6.0 | 6.5 | 7.0 |
| Water-containing polyol: | | | | | |
| Upon expiration of 7 days | | | | | |
| Gel time (min) | 10.5 | 4.0 | 5.5 | 4.0 | 4.0 |
| Tack-free time (min) | 16.0 | 6.0 | 8.0 | 7.0 | 7.5 |
| Storage stability | ○ | ○ | ○ | ○ | ○ |

**[Table 12]**

| | Evaluation Example 11 | Evaluation Example 12 | Evaluation Example 13 | Evaluation Example 14 |
|---|---|---|---|---|
| Corresponding Example/Comparative Example | Example 11 | Example 12 | Example 13 | Example 14 |
| Catalyst composition | Catalyst 11A | Catalyst 12A | Catalyst 13A | Catalyst 14A |
| Weight (mg) | 252 | 266 | 284 | 251 |
| Dehydrated polyol | | | | |
| Gel time (min) | 1.8 | 1.5 | 2.0 | 2.5 |
| Tack-free time (min) | 3.0 | 2.5 | 4.0 | 4.5 |
| Water-containing polyol: | | | | |
| Immediate after preparation | | | | |
| Gel time (min) | 2.5 | 1.7 | 2.5 | 3.0 |
| Tack-free time (min) | 5.0 | 3.0 | 4.5 | 5.0 |
| Water-containing polyol: | | | | |
| Upon expiration of 7 days | | | | |
| Gel time (min) | 2.7 | 1.8 | 3.0 | 3.5 |
| Tack-free time (min) | 5.0 | 3.5 | 6.0 | 6.5 |
| Storage stability | ○ | ○ | ○ | ○ |

As shown in Evaluation Examples 1 to 14, the catalyst compositions of the present invention underwent no substantial change in the reactivity even when stored for 7 days under a condition heated at 50°C in a water-containing polyol, and thus they can be said to be excellent in the preservation stability (storage stability).

### EVALUATION EXAMPLES 15 to 30 <Evaluation of water resistance>

Evaluation Examples 15 to 30 (corresponding to Comparative Examples 1 to 16, respectively) were carried out in the same manner as in Evaluation Example 1 except that the catalyst composition and the amount added were changed as shown in Tables 13 to 15. The results are also shown in Tables 13 to 15.

**[Table 13]**

| | Evaluation Example 15 | Evaluation Example 16 | Evaluation Example 17 | Evaluation Example 18 | Evaluation Example 19 | Evaluation Example 20 |
|---|---|---|---|---|---|---|
| Corresponding Example/Comparative Example | Comparative Example 1 | Comparative Example 2 | Comparative Example 3 | Comparative Example 4 | Comparative Example 5 | Comparative Example 6 |
| Catalyst composition | Catalyst 1B | Catalyst 2B | Catalyst 3B | Catalyst 4B | Catalyst 5B | Catalyst 6B |
| Weight (mg) | 152 | 168 | 168 | 223 | 200 | 217 |
| Dehydrated polyol | | | | | | |
| Gel time (min) | 1.8 | 1.8 | 1.8 | 2.3 | 2.5 | 1.5 |
| Tack-free time (min) | 3.0 | 3.0 | 3.0 | 3.5 | 4.0 | 3.5 |
| Water-containing polyol: | | | | | | |
| Immediate after preparation | | | | | | |
| Gel time (min) | 8.5 | 8.0 | 5.0 | 7.7 | 11.8 | 4.5 |
| Tack-free time (min) | 12.0 | 12.0 | 7.5 | 12.0 | 17.0 | 7.5 |
| Water-containing polyol: | | | | | | |
| Upon expiration of 7 days | | | | | | |
| Gel time (min) | 24.5 | 30.0 | 13.0 | 26.0 | 25.0 | 11.0 |
| Tack-free time (min) | 38.5 | 48.0 | 21.0 | 40.0 | 35.0 | 17.0 |
| Storage stability | × | × | × | × | × | × |

**[Table 14]**

| | Evaluation Example 21 | Evaluation Example 22 | Evaluation Example 23 | Evaluation Example 24 | Evaluation Example 25 | Evaluation Example 26 |
|---|---|---|---|---|---|---|
| Corresponding Example/Comparative Example | Comparative Example 7 | Comparative Example 8 | Comparative Example 9 | Comparative Example 10 | Comparative Example 11 | Comparative Example 12 |
| Catalyst composition | Catalyst 7B | Catalyst 8B | Catalyst 9B | Catalyst 10B | Catalyst 11B | Catalyst 12B |
| Weight (mg) | 279 | 187 | 152 | 251 | 202 | 143 |
| Dehydrated polyol | | | | | | |
| Gel time (min) | 1.8 | 2.7 | 10.0 | 1.8 | 2.0 | 2.5 |
| Tack-free time (min) | 3.0 | 4.5 | 16.5 | 3.0 | 3.0 | 3.5 |
| Water-containing polyol: | | | | | | |
| Immediate after preparation | | | | | | |
| Gel time (min) | 7.5 | 7.0 | 16.0 | 8.5 | 4.5 | 5.0 |
| Tack-free time (min) | 12.0 | 11.0 | 26.0 | 13.5 | 9.0 | 9.5 |
| Water-containing polyol: | | | | | | |
| Upon expiration of 7 days | | | | | | |
| Gel time (min) | 19.0 | 18.0 | 35.0 | 34.0 | 11.5 | 16.0 |
| Tack-free time (min) | 34.0 | 32.5 | 54.0 | 54.5 | 24.0 | 32.5 |
| Storage stability | × | × | × | × | × | × |

**[Table 15]**

| | Evaluation Example 27 | Evaluation Example 28 | Evaluation Example 29 | Evaluation Example 30 |
|---|---|---|---|---|
| Corresponding Example/Comparative Example | Comparative Example 13 | Comparative Example 14 | Comparative Example 15 | Comparative Example 16 |
| Catalyst composition | Catalyst 13B | Catalyst 14B | Catalyst 15B | Catalyst 16B |
| Weight (mg) | 50 | 50 | 255 | 298 |
| Dehydrated polyol | | | | |
| Gel time (min) | 10.5 | 4.5 | 1.3 | 2.3 |
| Tack-free time (min) | 17.0 | 9.0 | 2.5 | 3.5 |
| Water-containing polyol: | | | | |
| Immediate after preparation | | | | |
| Gel time (min) | 24.0 | 15.7 | 5.3 | 9.0 |
| Tack-free time (min) | 36.0 | 28.0 | 9.5 | 16.0 |
| Water-containing polyol: | | | | |
| Upon expiration of 7 days | | | | |
| Gel time (min) | At least 60 | 39.0 | 33.0 | 35.5 |
| Tack-free time (min) | At least 60 | At least 60 | 52.0 | 55.0 |
| Storaqe stability | × | × | × | × |

As shown in Evaluation Examples 15 to 26 (corresponding to Comparative Examples 1 to 12, respectively), the catalyst compositions wherein an iron compound and an alkanolamine compound having no TEDA skeleton in its structure were used, underwent substantial deterioration in the reactivity when stored for 7 days at 50°C.

As shown in Evaluation Examples 27 to 28 (corresponding to Comparative Examples 13 to 14, respectively), the catalyst compositions wherein only an iron compound was used, underwent substantial deterioration in the reactivity when stored for 7 days at 50°C.

As shown in Evaluation Examples 29 to 30 (corresponding to Comparative Examples 15 to 16, respectively), the catalyst compositions wherein an iron compound and TEDA (containing no hydroxy group in its molecule) were used, underwent substantial deterioration in the reactivity when stored for 7 days at 50°C.

From the foregoing results, it is evident that each of the catalyst compositions in Evaluation Examples 15 to 30 (corresponding to Comparative Examples 1 to 16) cannot be said to have good storage stability.

### EVALUATION EXAMPLE 31 <Evaluation of stability in water-blown system>

Polyols, water, surfactant and 0.16 part by weight of catalyst 1A (0.05 wt% to polyols as calculated as iron compound) were mixed in the raw material blend ratio as shown in Table 16 to prepare a polyol premix. 85.0 g of the polyol premix was taken into a 300 ml polyethylene cup and adjusted to a temperature of 25°C. A polyisocyanate mixture of T80 (TDI)/MR-200 (MDI) = 80/20 (weight ratio) having the temperature adjusted to 25°C in a separate container (each of T80 and MR-200 was manufactured by Nippon Polyurethane Industry Co., Ltd.), was put into the cup of the premix A in such an amount that the isocyanate index would be 105 and quickly stirred at 5,000 rpm for 5.0 seconds by a stirrer. The mixed and stirred liquid was transferred to a 2 L polyethylene cup having the temperature adjusted to 60°C, and the reactivity during foaming was measured.

### [Measured items for reactivity]

Cream time: The time until foam starts to rise is measured by visual observation.

Gel time: The time for change from a liquid state to a resinous state as the reaction proceeds, is measured.

In order to evaluate the storage stability, the polyol premix was stored in an oven having the temperature adjusted to 40°C, and after 7 days and 14 days, a similar evaluation was carried out. The results are shown in Table 17.

**[Table 16]**

| | Parts by weight (pbw) |
|---|---|
| Polyol A ¹⁾ | 60.0 |
| Polyol B ²⁾ | 40.0 |
| Water | 3.0 |
| Triethanolamine | 4.0 |
| Surfactant ³⁾ | 0.7 |
| Catalyst 1 ⁴⁾ | Varied |
| Catalyst 2 ⁵⁾ | 0.10 |

| | |
|---|---|
| 1) FA703: Polyether polyol manufactured by Sanyo Chemical Industries, Ltd. (OH value = 32.5 mgKOH/g) 2) KC900: Polymer polyol manufactured by Sanyo Chemical Industries, Ltd. (OH value = 20.5 mgKOH/g) 3) SRX274C: manufactured by Dow Corning Toray Co., Ltd. 4) Catalyst in Example or Comparative Example 5 Toyocat-ETS: Foaming catalyst manufactured by Tosoh Corporation, bis(2-dimethylaminoethyl) ether | |

**[Table 17]**

| | Evaluation Example 31 | Evaluation Example 32 | Evaluation Example 33 | Evaluation Example 34 |
|---|---|---|---|---|
| Corresponding Example/ Comparative Example | Example 1 | Example 2 | Example 9 | Example 11 |
| Catalyst composition | Catalyst 1A | Catalyst 2A | Catalyst 9A | Catalyst 11A |
| Parts by weight (pbw) | 0.16 | 0.27 | 0.28 | 0.25 |
| Immediate after preparation | | | | |
| Cream time (sec) | 12 | 12 | 12 | 12 |
| Gel time (sec) | 60 | 60 | 62 | 67 |
| Upon expiration of 7 days (40°C) | | | | |
| Cream time (sec) | 12 | 12 | 12 | 12 |
| Gel time (sec) | 62 | 60 | 62 | 63 |
| Upon expiration of 14 days (40°C) | | | | |
| Cream time (sec) | 12 | 12 | 12 | 12 |
| Gel time (sec) | 65 | 61 | 63 | 63 |
| Storage stability | ○ | ○ | ○ | ○ |

### EVALUATION EXAMPLES 32 to 34 <Evaluation of stability in water-blown system>

A polyol premix was prepared in accordance with Table 16 in the same manner except that the catalyst composition and the amount added, were changed as shown in Table 17, and evaluated in the same manner as in Evaluation Example 31. The results are also shown in Table 17.

As shown in Evaluation Examples 31 to 34, the catalyst compositions of the present invention underwent no substantial change in the reactivity even when stored under a condition heated at 40°C, and their storage stability can be said to be excellent. EVALUATION EXAMPLES 35 to 38 <Evaluation of stability in water-blown system>

A polyol premix was prepared in accordance with Table 16 in the same manner except that the catalyst composition and the amount added, were changed as shown in Table 18, and evaluated in the same manner as in Evaluation Example 31. The results are shown in Table 18.

**[Table 18]**

| | Evaluation Example 35 | Evaluation Example 36 | Evaluation Example 37 | Evaluation Example 38 |
|---|---|---|---|---|
| Corresponding Example/Comparative Example | Comparative Example 1 | Comparative Example 6 | Comparative Example 7 | Comparative Example 12 |
| Catalyst composition | Catalyst 1B | Catalyst 6B | Catalyst 7B | Catalyst 12B |
| Parts by weight (pbw) | 0.15 | 0.22 | 0.28 | 0.30 |
| Immediate after preparation | | | | |
| Cream time (sec) | 12 | 12 | 12 | 12 |
| Gel time (sec) | 66 | 67 | 69 | 70 |
| Upon expiration of 7 days (40°C) | | | | |
| Cream time (sec) | 12 | 12 | 12 | 12 |
| Gel time (sec) | Defoamed | Defoamed | Defoamed | Defoamed |
| Upon expiration of 14 days (40°C) | | | | |
| Cream time (sec) | - | - | - | - |
| Gel time (sec) | - | - | - | - |
| Storage stability | × | × | × | × |

As shown in Evaluation Examples 35 to 38, the catalyst compositions wherein an iron compound and a tertiary amine other than an alkanolamine having a TEDA skeleton, were used, underwent deterioration in the gelling activity during the storage under heating for 7 days, whereby formation of foam was impossible, and thus, their storage stability cannot be said to be good.

### INDUSTRIAL APPLICABILITY

The catalyst composition of the present invention is useful for the production of a polyurethane resin.

## Claims

1. A catalyst composition for polyurethane resin production, which comprises an iron compound represented by the following formula (1) and a tertiary amine compound represented by the following formula (3):
Feⁿ⁺(L)ₙ (1)
in the formula (1), each L independently is a β-diketonate ligand represented by the following formula (2), and n is 2 or 3, 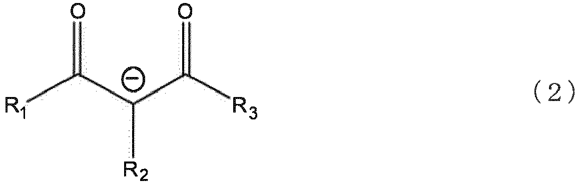 in the formula (2), each of R₁ and R₃ which are independent of each other, is a C₂₋₁₀ alkyl group, and R₂ is a hydrogen atom or a C₁₋₇ alkyl group, 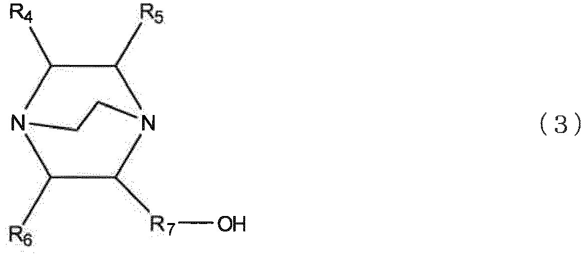 in the formula (3), each of R₄ to R₆ which are independent of one another, is a hydrogen atom or a C₁₋₁₀ alkyl group, and R₇ is a C₁₋₁₀ alkylene group.

2. The catalyst composition for polyurethane resin production according to Claim 1, wherein in the β-diketonate ligand represented by the formula (2), R₂ is a hydrogen atom.

3. The catalyst composition for polyurethane resin production according to Claim 1 or 2, wherein the β-diketonate ligand represented by the formula (2) is at least one member selected from the group consisting of 3,5-heptanedionate, 3,5-octanedionate, 2-methyl-3,5-heptanedionate, 2-methyl-3,5-nonanedionate and 2,6-dimethyl-3,5-heptanedionate.

4. The catalyst composition for polyurethane resin production according to any one of Claims 1 to 3, wherein in the tertiary amine compound represented by the formula (3), R₇ is a methylene group or an ethylene group.

5. The catalyst composition for polyurethane resin production according to any one of Claims 1 to 4, wherein the tertiary amine compound represented by the formula (3) is 2-hydroxymethyl-1, 4-diazabicyclo[2.2.2]octane or 2-hydroxyethyl-1,4-diazabicyclo[2.2.2]octane.

6. The catalyst composition for polyurethane resin production according to any one of Claims 1 to 5, which contains at least 1 mol and at most 50 mol of the tertiary amine compound represented by the formula (3) relative to 1 mol of the iron compound represented by the formula (1).

7. A method for producing a polyurethane resin, which comprises reacting a polyol component and an organic polyisocyanate component in the presence of the catalyst composition for polyurethane resin production as defined in any one of Claims 1 to 6.

8. A method for producing a polyurethane resin, which comprises reacting a polyol component and an organic polyisocyanate component in the presence of the catalyst composition for polyurethane resin production as defined in any one of Claims 1 to 6 and a blowing agent.

9. A method for producing a polyurethane resin, which comprises reacting a polyol premix comprising the catalyst composition for polyurethane resin production as defined in any one of Claims 1 to 6, a polyol component and a blowing agent, with an organic polyisocyanate component.

10. The method for producing a polyurethane resin according to Claim 8 or 9, wherein the blowing agent contains water.

## Patentansprüche

1. Katalysatorzusammensetzung für die Polyurethanharzherstellung, die eine Eisenverbindung der folgenden Formel (1) und eine tertiäre Aminverbindung der folgenden Formel (3) umfasst:
Feⁿ⁺(L)ₙ (1)
in der Formel (1) ist jedes L unabhängig voneinander ein β-Diketonat-Ligand, dargestellt durch die folgende Formel (2), und n ist 2 oder 3, 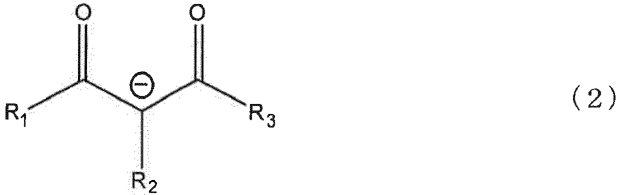 in der Formel (2) ist jedes R₁ und R₃, die unabhängig voneinander sind, eine C₂₋₁₀-Alkylgruppe, und R₂ ist ein Wasserstoffatom oder eine C₁₋₇-Alkylgruppe, 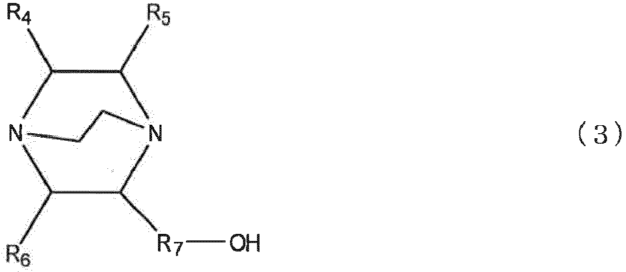 in der Formel (3) ist jedes R₄ bis R₆, die voneinander unabhängig sind, ein Wasserstoffatom oder eine C₁₋₁₀-Alkylgruppe, und R₇ ist eine C₁₋₁₀-Alkylengruppe.

2. Katalysatorzusammensetzung für die Polyurethanharzherstellung nach Anspruch 1, wobei in dem β-Diketonat-Liganden, dargestellt durch die Formel (2), R₂ ein Wasserstoffatom ist.

3. Katalysatorzusammensetzung für die Polyurethanharzherstellung nach Anspruch 1 oder 2, wobei der β-Diketonat-Ligand der Formel (2) mindestens ein Element ist, das aus der Gruppe bestehend aus 3,5-Heptandionat, 3,5-Octandionat, 2-Methyl-3,5-heptandionat, 2-Methyl-3,5-nonandionat und 2,6-Dimethyl-3,5-heptandionat ausgewählt wird.

4. Katalysatorzusammensetzung für die Polyurethanharzherstellung nach einem der Ansprüche 1 bis 3, wobei in der tertiären Aminverbindung, dargestellt durch die Formel (3), R₇ eine Methylengruppe oder eine Ethylengruppe ist.

5. Katalysatorzusammensetzung für die Polyurethanharzherstellung nach einem der Ansprüche 1 bis 4, wobei die tertiäre Aminverbindung, dargestellt durch die Formel (3), 2-Hydroxymethyl-1,4-Diazabicyclo[2.2.2]octan oder 2-Hydroxyethyl-1,4-diazabicyclo[2.2.2]octan ist.

6. Katalysatorzusammensetzung für die Polyurethanharzherstellung nach einem der Ansprüche 1 bis 5, die mindestens 1 Mol und höchstens 50 Mol der tertiären Aminverbindung, dargestellt durch die Formel (3), bezogen auf 1 Mol der Eisenverbindung der Formel (1), enthält.

7. Verfahren zur Herstellung eines Polyurethanharzes, das das Umsetzen einer Polyolkomponente und einer organischen Polyisocyanatkomponente in Gegenwart der Katalysatorzusammensetzung für die Polyurethanharzherstellung, wie in einem der Ansprüche 1 bis 6 definiert, umfasst.

8. Verfahren zur Herstellung eines Polyurethanharzes, das die Umsetzung einer Polyolkomponente und einer organischen Polyisocyanatkomponente in Gegenwart der Katalysatorzusammensetzung für die Polyurethanharzherstellung nach einem der Ansprüche 1 bis 6 und eines Treibmittels, umfasst.

9. Verfahren zur Herstellung eines Polyurethanharzes, das das Umsetzen einer Polyolvormischung, umfassend die Katalysatorzusammensetzung für die Polyurethanharzherstellung nach einem der Ansprüche 1 bis 6, eine Polyolkomponente und ein Treibmittel, mit einer organischen Polyisocyanatkomponente umfasst.

10. Verfahren zur Herstellung eines Polyurethanharzes nach Anspruch 8 oder 9, wobei das Treibmittel Wasser enthält.

## Revendications

1. Composition de catalyseur pour la production de résine de polyuréthane, qui comprend un composé du fer représenté par la formule (1) suivante et un composé amine tertiaire représenté par la formule (3) suivante :
Feⁿ⁺(L)ₙ (1)
dans la formule (1), chaque L est indépendamment un ligand β-dicétonate représenté par la formule (2) suivante, et n vaut 2 ou 3, 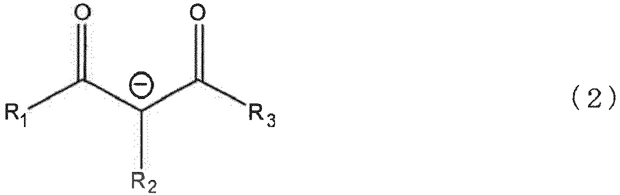 dans la formule (2), chacun de R₁ et R₃, qui sont indépendants l'un de l'autre, est un groupe alkyle en C₂ à C₁₀, et R₂ est un atome d'hydrogène ou un groupe alkyle en C₁ à C₇, 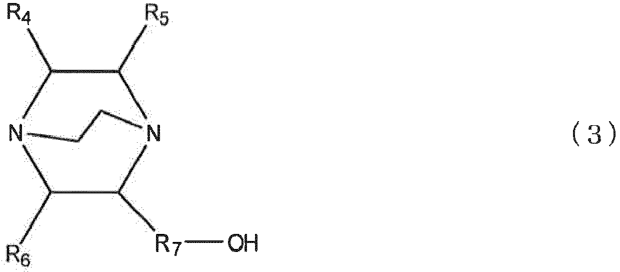 dans la formule (3), chacun de R₄ à R₆, qui sont indépendants les uns des autres, est un atome d'hydrogène ou un groupe alkyle en C₁ à C₁₀, et R₇ est un groupe alkylène en C₁ à C₁₀.

2. Composition de catalyseur pour la production de résine de polyuréthane selon la revendication 1, dans laquelle, dans le ligand β-dicétonate représenté par la formule (2), R₂ est un atome d'hydrogène.

3. Composition de catalyseur pour la production de résine de polyuréthane selon la revendication 1 ou 2, dans laquelle le ligand β-dicétonate représenté par la formule (2) est au moins un membre choisi dans l'ensemble constitué par 3,5-heptanedionate, 3,5-octanedionate, 2-méthyl-3,5-heptanedionate, 2-méthyl-3,5-nonanedionate et 2,6-diméthyl-3,5-heptanedionate.

4. Composition de catalyseur pour la production de résine de polyuréthane selon l'une quelconque des revendications 1 à 3, dans laquelle, dans le composé amine tertiaire représenté par la formule (3), R₇ est un groupe méthylène ou un groupe éthylène.

5. Composition de catalyseur pour la production de résine de polyuréthane selon l'une quelconque des revendications 1 à 4, dans laquelle le composé amine tertiaire représenté par la formule (3) est le 2-hydroxyméthyl-1,4-diazabicyclo[2.2.2]octane ou le 2-hydroxyéthyl-1,4-diazabicyclo[2.2.2]octane.

6. Composition de catalyseur pour la production de résine de polyuréthane selon l'une quelconque des revendications 1 à 5, qui contient au moins 1 mole et au plus 50 moles du composé amine tertiaire représenté par la formule (3) par mole du composé du fer représenté par la formule (1).

7. Procédé pour produire une résine de polyuréthane, qui comprend la réaction d'un composant polyol et d'un composant polyisocyanate organique en présence de la composition de catalyseur pour la production de résine de polyuréthane telle que définie dans l'une quelconque des revendications 1 à 6.

8. Procédé pour produire une résine de polyuréthane, qui comprend la réaction d'un composant polyol et d'un composant polyisocyanate organique en présence de la composition de catalyseur pour la production de résine de polyuréthane telle que définie dans l'une quelconque des revendications 1 à 6 et d'un agent d'expansion.

9. Procédé pour produire une résine de polyuréthane, qui comprend la réaction d'un prémélange de polyol comprenant la composition de catalyseur pour la production de résine de polyuréthane telle que définie dans l'une quelconque des revendications 1 à 6, un composant polyol et un agent d'expansion, avec un composant polyisocyanate organique.

10. Procédé pour produire une résine de polyuréthane selon la revendication 8 ou 9, dans lequel l'agent d'expansion contient de l'eau.
